## Europäisches Patentamt

## European Patent Office

## Office européen des brevets

(11) Veröffentlichungsnummer: **0 062 913 B1**

(12) **EUROPÄISCHE PATENTSCHRIFT**

(45) Veröffentlichungstag der Patentschrift:
17.09.86

(21) Anmeldenummer: **82103060.8**

(22) Anmeldetag: **08.04.82**

(51) Int. Cl.⁴: **A 61 M 5/14**, A 61 M 5/16, A 61 M 1/14

(54) **Tropfkammer.**

(30) Priorität: **15.04.81 DE 3115299**

(43) Veröffentlichungstag der Anmeldung:
**20.10.82 Patentblatt 82/42**

(45) Bekanntmachung des Hinweises auf die Patenterteilung:
**17.09.86 Patentblatt 86/38**

(84) Benannte Vertragsstaaten:
**BE FR GB IT NL**

(56) Entgegenhaltungen:
**DE - A - 2 851 838**
**GB - A - 2 000 685**
**GB - A - 2 028 976**
**US - A - 3 042 038**
**US - A - 4 177 149**

(73) Patentinhaber: **Fresenius AG, Gluckensteinweg 5,
D-6380 Bad Homburg (DE)**

(72) Erfinder: **Mathieu, Bernd, Dr. rer. nat., Am Köppchen 16a,
D-6683 Spiesen (DE)**

(74) Vertreter: **Luderschmidt, Wolfgang, Dr. Dipl-Chem.,
Sonnenberger Strasse 100, D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft eine Tropfkammer gemäss dem Oberbegriff des Anspruchs 1.

Aus der US-A-3 042 038 ist eine Vorrichtung der eingangs erwähnten Art bekannt, die zur Abtrennung von Luft bei der Infusion von parenteralen Flüssigkeiten eingesetzt wird. Dabei wird die zu infundierende Flüssigkeit durch eine Zuführungsöffnung in den Innenraum der Trennkammer eingeführt, innerhalb der ein Schwimmer angeordnet ist, der bei Füllung der Kammer die Entlüftungsöffnung des Entlüftungsrohrs verschliesst. Im Boden der Kammer ist ein Ablaufrohr vorgesehen, durch das die Flüssigkeit abfliessen kann, wobei die Strömungsgeschwindigkeit der abfliessenden Flüssigkeit durch ein Drosselventil reguliert werden kann.

Diese bekannte Trennkammer lässt sich nicht zufriedenstellend für medizinische Zwecke, insbesondere nicht für die Hämodialyse einsetzen. Dies ist darauf zurückzuführen, dass die Entlüftungsöffnung unmittelbar mit der Umwelt in Verbindung steht, so dass grundsätzlich mit einer Verkeimung des Entlüftungsrohrs und insbesondere des Innenraums der Trennkammer während der Behandlungszeit gerechnet werden muss. Weiterhin besteht die Gefahr, dass der Schwimmer durch gerinnendes Blut an der Gehäusewand oder an dem Entlüftungsrohr festklebt mit der Folge, dass die Funktion der Trennkammer aufgehoben wird. Demzufolge besteht also die Gefahr, dass das zugeführte Blut in die Umgebung verspritzt werden kann oder dass Luft bei verschlossener Entlüftungsöffnung in das Gefässsystem des Patienten gedrückt werden kann, was zu lebensgefährlichen Zuständen führt.

Aus der DE-A-2 851 838 ist ein Infusionsfilter bekannt, in dem Luft aus einer wässrigen Lösung entfernt wird. Hierzu sind sowohl ein hydrophiles als auch ein hydrophobes Filter vorgesehen, wobei das hydrophile Filter als Sperre gegen Luft dient und das hydrophobe Filter als Sperre für die Flüssigkeit vorgesehen ist. Derartige Filter eignen sich nicht als Tropfkammer für die Hämodialyse, da die hydrophile Filterschicht, die die Abtrennung des in der zugeführten Flüssigkeit enthaltenen Gases bewirken soll, die Blutpassage verhindert. Darüber hinaus dient dieser bekannte Infusionsfilter einem völlig anderen Zweck als eine Tropfkammer.

Demgegenüber liegt der Erfindung die Aufgabe zugrunde, die Tropfkammer der eingangs erwähnten Art so vorzubilden, dass sie selbsttätig ihr Flüssigkeitsniveau unter Aufrechterhaltung der Sterilität im wesentlichen konstant hält.

Die Lösung der Aufgabe erfolgt dadurch, dass die zweite Öffnung zur Waagrechten geneigt angeordnet ist und dass die Mittel zum Verschliessen dieser zweiten Öffnung eine flüssigkeitsabstossende, gasdurchlässige Membran ist, die die zweite Öffnung bedeckt.

Erfindungsgemäss wird eine Tropfkammer zur Verfügung gestellt, die sich selbsttätig während der Dialysebehandlung entlüftet, ohne dass die Gefahr besteht, dass die Sterilität innerhalb der Dialysekammer aufgehoben wird. Darüber hinaus besteht weiterhin nicht die Gefahr, dass durch die Entlüftungsöffnung Blut austritt, da die hydrophobe Membran, die die Entlüftungsöffnung verdeckt, durch Blut nicht benetzbar ist und somit das Blut vor dem Austritt zurückhält.

Insofern ist nach der Startperiode der Dialysevorrichtung, d.h. nach der Einstellung des Blutniveaus in der Tropfkammer kein weiterer Bedienungshandgriff mehr notwendig.

Da das Blut grundsätzlich mit Überdruck in die Tropfkammer eingeführt wird, ist normalerweise davon auszugehen, dass sich der Blutpegel in Höhe der hydrophoben, luftdurchlässigen Membran des Entlüftungsrohrs einstellt. Dabei wird ein Luftpolster zwischen der Spitze des Entlüftungsrohrs und dem Deckel eingeschlossen, der als Druckpuffer dient. Dies hat den Vorteil, dass Pulsationen des Blutstromes ausgeglichen werden können.

Die Entfernung der Luft aus dem Blut erfolgt an der hydrophoben Membran zwangsläufig, da der in der Kammer vorliegende Überdruck die Luft durch die Poren der hydrophoben Membran presst. Anderseits reicht jedoch dieser Überdruck nicht aus, um die wässrige Flüssigkeit durch die Poren der Membran zu drücken. Infolgedessen ist bei der erfindungsgemässen Tropfkammer sichergestellt, dass an der mit der hydrophoben Membran versehenen Entlüftungsöffnung lediglich Luft, nicht jedoch Flüssigkeit austreten kann.

Diese selbsttätige Regelung der Luftausscheidung in einer Dialysevorrichtung hat zur Folge, dass die häufig während der Nacht durchgeführte Dialysebehandlung eines Patienten in der Regel ohne Alarm verläuft, da das Luftpolster in der Tropfkammer nicht mehr über eine einmal eingestellte Höhe ansteigen kann. Somit kann sich der Patient in Ruhe der Dialysebehandlung unterziehen und wird nicht durch wiederholte Alarmphasen gestört.

Weiterhin entfällt durch die selbsttätige Regulierung der Flüssigkeitshöhe und des Luftpolsters ein weiterer, für Laien nicht einfach zu handhabender Bedienungsschritt, so dass die Bedienbarkeit der Dialysevorrichtung verbessert wird.

Weitere Vorteile der Erfindung ergeben sich aus der nachfolgenden Beschreibung anhand der in der Zeichnung gezeigten Ausführungsbeispiele. Es zeigen:

Fig. 1 eine schematische Ansicht einer Dialysevorrichtung unter nicht massstabsgetreuer Hervorhebung der erfindungsgemässen Tropfkammer,

Fig. 2a eine erste Ausführungsform der erfindungsgemässen Tropfkammer im Längsschnitt,

Fig. 2b eine zweite Ausführungsform der erfindungsgemässen Tropfkammer im Längsschnitt,

Fig. 3 eine erste Ausführungsform einer Membrananordnung im Querschnitt, die in einer der Tropfkammern gemäss Fig. 2 einsetzbar ist,

Fig. 4 eine zweite Ausführungsform einer Membrananordnung im Querschnitt, die ebenfalls in einer der in Fig. 2 gezeigten Tropfkammern einsetzbar ist und

Fig. 5 eine dritte Ausführungsform einer Tropfkammer im Längsschnitt.

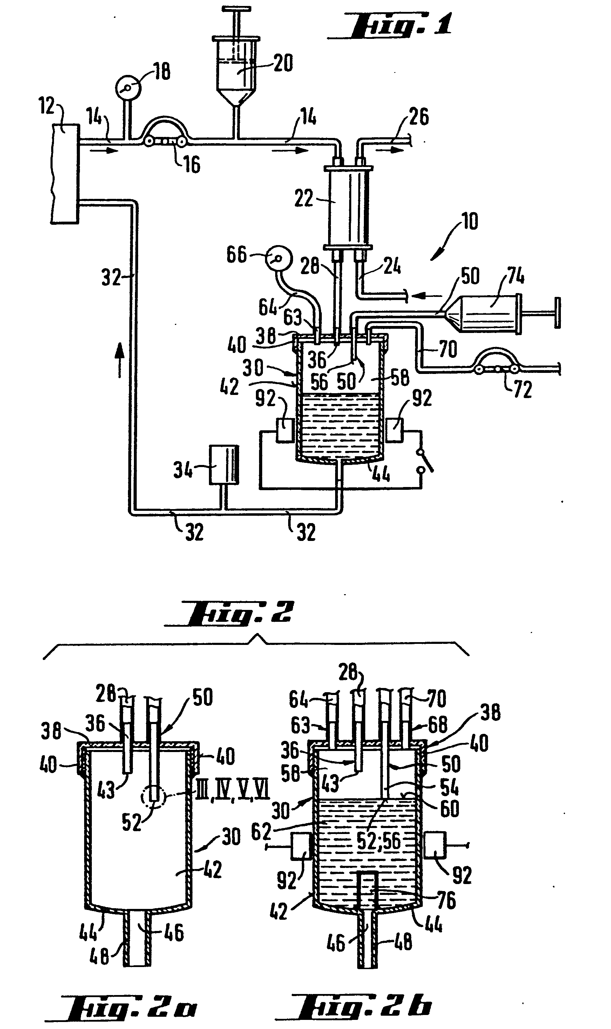

In Fig. 1 ist schematisch eine Dialysevorrichtung 10 gezeigt, die üblicherweise zur Hämodialyse von Blut eingesetzt wird. Bei dieser Hämodialyse wird arteriell aus dem Patienten 12 über eine Leitung 14 Blut entnommen, wobei eine peristaltische Pumpe 16, üblicherweise eine Schlauchpumpe diese Blutentnahme unterstützt. Hierdurch entsteht in der Leitung 14 ein Unterdruck im Bereich von etwa 100 mbar, der durch ein Druckmanometer 18 angezeigt werden kann. Stromab der peristaltischen Pumpe 16 kann über eine Spritze 20 eine Hilfsflüssigkeit, beispielsweise Heparin, dem Blut zugeführt werden, um beispielsweise die Blutgerinnung zu hemmen. Die Leitung 14 ist hinter der Spritze 20 mit dem Dialysator 22 verbunden. Dieser Dialysator 22 enthält eine nicht dargestellte semipermeable Dialysemembran, an deren einen Seite das Blut über die Leitung 14 vorbeigeführt wird, während auf ihrer anderen Seite eine Spülflüssigkeit, die aus einer gewöhnlichen Kochsalzlösung mit gewissen medizinischen Zusätzen bestehen kann, über die Leitung 24 zugeführt und über die Leitung 26 entfernt wird.

Der blutseitige Ausgang des Dialysators 22 ist über die Leitung 28 mit der erfindungsgemässen Tropfkammer 30 verbunden, so dass das Blut direkt in diese Tropfkammer 30 geführt wird, wenn es den Dialysator 22 durchlaufen hat.

Nach dem Durchlaufen der Tropfkammer 30 wird das Blut über die Leitung 32, die gegebenenfalls eine spezielle Luftabscheidevorrichtung 34 enthalten kann, zum Patienten 12 zurückgeführt.

Es ist anzumerken, dass auf der arteriellen Seite der Blutführung ein Überdruck von ca. 100 mbar vorliegt, der auf die Wirkung der Pumpe 16 zurückzuführen ist. Diese Pumpe 16 kann auf ihrer Saugseite natürlich auch etwas Luft ansaugen, die in der Dialysevorrichtung 10 selbst entfernt werden muss, um eine Luftembolie des Patienten zu unterdrücken. Die Überwachung des Luftzuflusses und Einstellung des Blutzuflusses lässt sich vorteilhaft in der Tropfkammer 30 durchführen, die nachstehend unter Bezugnahme auf die Fig. 1 in Verbindung mit Fig. 2 erläutert wird.

Das gereinigte Blut wird, wie vorstehend festgestellt, über die Leitung 28 der Tropfkammer 30 zugeführt, die vorteilhafterweise einen Nippel 36 aufweist, der zum Anschluss der Leitung 28 dient. Dieser Nippel 36 befindet sich auf einer Kappe 38, deren Umfangsränder 40 den die Kammer bildenden Hohlkörper 42 gasdicht umfassen. Die Kappe 38 kann jedoch auch als ebener Deckel ausgebildet sein, der gasdicht auf den Hohlkörper 42 aufgesetzt ist. Um eine gasdichte Verbindung der Kappe 38 mit dem Hohlkörper 42 zu erhalten, können diese miteinander verklebt oder verschmolzen sein.

Der Hohlkörper 42 selbst, der vorteilhafterweise zylindrisch ausgebildet ist, weist einen Boden 44 auf, der vorteilhafterweise sich kegelförmig nach unten verjüngt. Dieser Boden 44 wird von der Öffnung 46 eines Abzugsnippels 48 durchsetzt, der an die Leitung 32 angeschlossen werden kann.

Der Nippel 36 durchsetzt die Kappe 38 und ragt mit seiner Spitze 40 etwas in den Hohlkörper 42. Aus dieser Spitze 43 strömt beim Betrieb der Dialysevorrichtung das Blut in den Hohlkörper 42 ein und steigt zunächst im Hohlkörper 42, wie in Fig. 2b gezeigt ist, hoch, da ein Weiterfliessen des Blutes durch die Öffnung 46 in die Leitung 32 zum Patienten durch den Strömungswiderstand darin verhindert wird.

In der Kappe 38 ist ein Entlüftungsrohr 50 vorgesehen, der diese Kappe durchsetzt, wobei dieses Entlüftungsrohr 50 mit seinem kammerseitigen Ende 52 weiter in den Hohlkörper 42 ragt als die Spitze 43 des Nippels 36. Vorteilhafterweise beträgt der Höhenunterschied zwischen dem kammerseitigen Ende 52 und der Spitze 43 etwa 0,5 bis 2,5 cm, insbesondere etwa 1,5 cm.

Das Entlüftungsrohr 50 ist natürlich wie die Nippel 36 und 48 innen hohl, so dass bei einem Ansteigen des Blutes in dem Hohlkörper 42 die sich im Hohlkörper 42 befindliche Luft durch ihn hindurch entweichen kann. Wäre das Entlüftungsrohr 50 nicht an seinem kammerseitigen Ende 52 verschlossen, so würde das Blut beim Erreichen des Endbereichs 54 des Entlüftungsrohrs 50 die Luftverdrängung beenden und selbst durch das Entlüftungsrohr 50 austreten, was unter allen Umständen verhindert werden muss.

Daher ist der als Umfangsrand ausgebildete Endbereich 54 mit einer Membran 56 versehen, die einerseits luftdurchlässig ist, anderseits jedoch hydrophob, d.h. wasserabstossend ist. Diese Membran 56 lässt also beim Befüllen des Hohlkörpers 42 solange die überschüssige Luft durch, bis das Blut, wie in Fig. 2b gezeigt ist, den Endbereich 54 des Entlüftungsrohrs 50 erreicht hat. Da die semipermeable Membran 56 kein Wasser, also auch nicht Blut durchlässt, wird das im Raum 58 eingeschlossene Luftvolumen solange elastisch komprimiert, bis der sich in dem entstandenen Luftpolster aufbauende Gegendruck grösser wird als der Strömungswiderstand in der Leitung 32. Dadurch wird ein druckausgeglichenes System geschaffen, bei dem das Niveau 60 des Blutspiegels nur geringfügig schwankt. Wenn nämlich durch geringfügige Mengen Luft das Luftpolster im Raum 58 anwächst und das Niveau 60 nach unten drückt, geht diese Absenkung nur bis zur Höhe der Membran 56, da dann anschliessend die Luft durch die Membran 56 entweichen kann. Somit entspricht die Fallhöhe des Blutes dem vorstehend genannten Höhenunterschied zwischen dem Endbereich 54 des Entlüftungsrohrs 50 und der Spitze 43 des Nippels 36. Diese Fallhöhe soll den vorstehend genannten Massen entsprechen, die gewährleisten, dass das Blut einerseits optisch gut überwacht werden kann, anderseits aber jedoch nicht zuviel Luft aus dem Raum 58 beim Fall mitreisst, was zu einer unerwünschten Luftblasenbildung im Blutraum 62 führen kann.

In Fig. 2b ist eine weitere Ausführungsform einer erfindungsgemässen Tropfkammer 30 gezeigt. Diese Tropfkammer 30 weist neben den bereits vorstehend genannten Nippeln 36, 48 und 50 einen weiteren Nippel 63 auf, der gemäss Fig. 1 über eine Leitung 64 mit einem Druckmanometer 66 verbunden sein kann. Dieses Druckmanometer 66 zeigt direkt den im Raum 58 befindlichen Luftdruck an und kann über eine nicht gezeigte Alarmvorrichtung Alarm auslösen, sofern der Druck übermässig ansteigt oder abfällt. Weiterhin kann die Tropfkammer 30 einen vierten Nippel 68 aufweisen, der über eine Leitung 70 mit einer Schlauchpumpe 72 und gegebenenfalls mit einer Spritze 74 verbunden sein kann. Die Schlauchpumpe 72, die vorteilhafterweise handbetrieben sein kann, dient zur rascheren Entfernung der Luft beim Anfahren und/oder während des Betriebs der Dialysevorrichtung 10. Durch diese Schlauchpumpe 72 kann somit Luft rasch entzogen oder aber auch zugeführt werden. Dies kann auch durch eine Spritze 74 erfolgen, die mit der Leitung 70 in Verbindung steht.

Jedoch ist der Einsatzbereich der Spritze 74 nicht auf die Luftentfernung beschränkt. Vielmehr kann über die Spritze 74 vorteilhafterweise auch eine Flüssigkeit zugeführt werden, die dem Blut beigemischt werden soll.

Die gemäss Fig. 2b gezeigte Ausführungsform weist ein am Boden 44 angeordnetes feines Sieb 76 auf, das zylinderförmig auf dem Boden 44 angeordnet ist und mit dem Umfangsrand der Öffnung 46 verbunden ist. Beim Abfluss muss das Blut somit das Sieb, dessen Maschenweite etwa 40 bis 200 µm beträgt, durchfliessen, so dass darin geronnene Teilchen hängenbleiben.

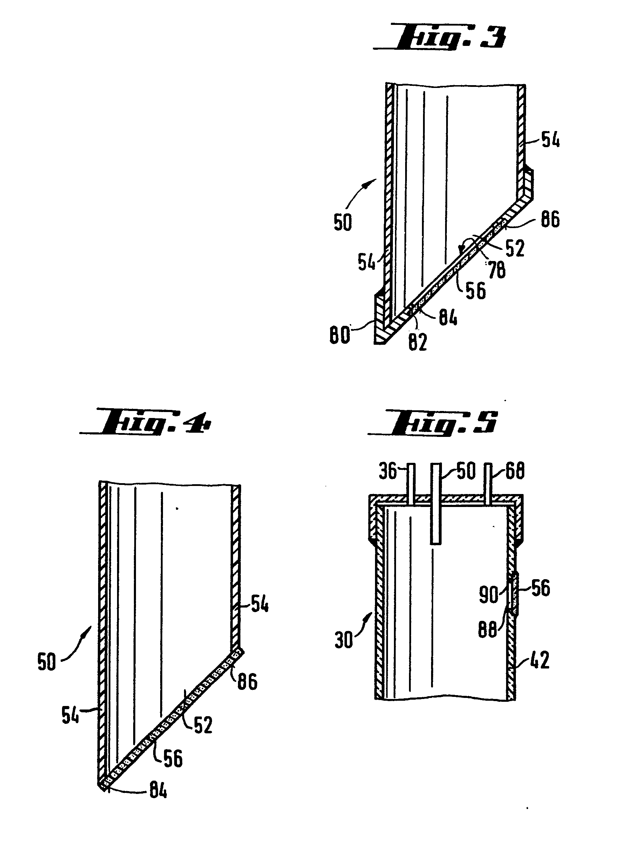

Nachstehend wird die Membrananordnung unter Bezugnahme auf Fig. 3 bis 5 beschrieben.

Gemäss Fig. 3 überspannt die Membran 56 die Öffnung 78 der Kappe 80 und ist entlang ihres Umfangs mit dem sich an die Öffnung 78 anschliessenden Randbereichs 82 der Kappe 80 vergossen. Die Kappe 80 selbst ist auf den Endbereich 54 des Entlüftungsrohrs 50 aufgesetzt und dort gas- und flüssigkeitsdicht befestigt, beispielsweise durch Verschweissen oder Verkleben.

Gemäss Fig. 4 ist die Membran 56 direkt auf dem Umfangsrand des Endbereichs 54 des Entlüftungsrohrs 50 befestigt, was wiederum durch Aufkleben oder Aufschweissen erfolgen kann.

Bei den in Fig. 3 und 4 gezeigten Anordnungen ist die durch den Umfangsrand des Endbereichs 54 des Entlüftungsrohrs 50 gebildete Ebene schräg zur Längsachse des Entlüftungsrohrs 50 angeordnet. Der von der Längsachse auf dieser Ebene gebildete Winkel liegt vorzugsweise in einem Bereich von 30 bis 60, insbesondere etwa 45°. Diese Schräganordnung hat den Vorteil, dass die Regulierung des Niveaus 60 nicht sofort gestoppt wird, wenn dieses Niveau 60 mit den Umfangsrändern des Endbereichs 54 zusammenfällt, sondern dass das Niveau 60 zuerst auf den unteren Schrägrand 84 auftrifft und allmählich an der Membran 56 hochsteigt. Erst wenn der obere Schrägrand 86 erreicht ist, wird die Luftausscheidung gestoppt, so dass auch geringe Überschussmengen an Luft auch mit dieser Anordnung entfernt werden können.

In Fig. 5 ist eine weitere Ausführungsform einer Tropfkammer 30 gezeigt, bei der der Hohlkörper 42 selbst eine seine Wand durchsetzende Öffnung 88 aufweist, die wiederum vollständig mit der Membran 56 bedeckt ist. Der Umfangsrand der Membran 56 ist mit dem Umfangsrand der Öffnung 88 fest verbunden, beispielsweise durch Verkleben oder Verschweissen. Der Abstand des oberen Öffnungsrandes 90 vom kammerseitigen Ende 52 bzw. dessen Endbereich 54 liegt wiederum im vorstehend genannten Bereich zwischen 0,5 bis 2,5 cm, vorzugsweise etwa 1,5 cm. Diese Beziehung gilt auch für den oberen Schrägrand 86 bei der Ausführungsform gemäss Fig. 3 und 4.

Als Material für die Tropfkammer wird vorteilhafterweise ein transparentes Material, insbesondere ein durchsichtiger Kunststoff mit elastischen Eigenschaften gewählt, bei dem eine gute optische Überwachung der Tropfkammer 30 gewährleistet ist. Als Kunststoffmaterialien kommen beispielsweise PVC, Polycarbonat, Polypropylen, Polyacryl-Glas, Polyethylen u.dgl. in Frage. Von diesen Materialien ist PVC bevorzugt.

Als Materialien für die Membran 56 kommen wasserundurchlässige, semipermeable Membranen auf Kunststoffbasis in Frage, die wenigstens bis zu einem Überdruck von 500 mbar wasserundurchlässig sind. Vorzugsweise beträgt diese Grenze wenigstens 1, insbesondere 1,5 bar. Bei einer einsetzbaren Membran wurde beispielsweise gemessen, dass Wasser aus Blut erst oberhalb 1,6 bar durchtritt, während reines Wasser bis 2,75 bar überhaupt nicht durchgetreten ist. Diese Drükke treten jedoch keinesfalls bei der Tropfkammer im Dialysebetrieb auf, so dass ein ausreichender Sicherheitsraum für diesen Betrieb vorhanden ist. Sollte tatsächlich eine Membran reissen, so kann durch Druckabfall im Druckmanometer 66 sofort Alarm gegeben werden.

Als Kunststoffmaterialien für derartige Membranen kommem organische Polymere, insbesondere PTFE (Teflon), Silikone, Polyethylen, Polypropylen, sowie hydrophobierte Polymere, beispielsweise silikonisierte Polymere, in Frage. Von diesen Polymeren ist PTFE bevorzugt.

Die semipermeable Eigenschaft ist auf eine mikroporöse Struktur der üblicherweise in Folienform mit einer Dicke von 0,01 bis 0,3, vorzugsweise 0,05 bis 0,1 mm vorliegenden Membran zurückzuführen. Diese mikroporöse Struktur wird entweder durch ein Kreuznetzwerk des polymerisierten Materials gebildet, wobei ein mittlerer Porendurchmesser erhalten wird, oder durch eine kanalförmige Anordnung einer Vielzahl von Löchern erzeugt. In der Regel liegt der Porendurchmesser dieser Öffnungen unter 1 µm, insbesondere unter 0,5 µm. Besonders bevorzugt ist eine Membran mit einem Porendurchmesser von etwa 0,2 µm.

Aus Gründen der Festigkeit kann die hydrophobe Membran auch durch eine Stützmembran abgestützt sein. Diese Stützmembran, deren Poren erheblich grösser sind als die der hydrophoben Membran kann ebenfalls aus einem Kunststoff, beispielsweise Polyester, Polyethylen, Polypropylen und dergleichen, hergestellt sein. In der erfindungsgemässen Tropfkammer wird eine derartige laminierte Membran blutseitig, d.h. in Richtung auf die Kammer angeordnet, während die Stützschicht nicht mit der wasserhaltigen Flüssigkeit in Berührung kommt. Vorteilhafterweise liegt die Dicke des Laminats in einem Bereich von 0,1 bis 0,3, insbesondere etwa 0,2 mm.

Die elastische Tropfkammer 30 kann in der Dialysevorrichtung 10 mit einer Überwachungsvorrichtung 92 verbunden werden, die das untere Niveau des Blutspiegels kontrolliert, so dass eine vollständige Regulierung des Blutniveauspiegels in der Tropfkammer 30 erreicht werden kann. Hierdurch wird eine grösstmögliche Sicherheit bei geringem apparativem Aufwand und einfacher Handhabbarkeit erreicht.

## Patentansprüche

1. Tropfkammer, insbesondere zur Verwendung in einer Hämodialysevorrichtung, aufweisend einen Hohlkörper (42) mit einem Boden (44) und einem Deckel (38), mit einer Zulauföffnung im Deckel (38) zur Einführung der zu fördernden Flüssigkeit, einer zweiten Öffnung (78, 88) zur Entlüftung und Mitteln (56) zum Verschliessen der zweiten Öffnung (78, 88) gegenüber der Flüssigkeit und mit einer Ablauföffnung (46) im Boden (44), dadurch gekennzeichnet, dass die zweite Öffnung (78, 88) zur Waagrechten geneigt angeordnet ist und dass das Mittel zum Verschliessen dieser zweiten Öffnung (78, 88) eine flüssigkeitsabstossende, gasdurchlässige Membran (56) ist, die die Öffnung (78, 88) bedeckt.

2. Tropfkammer nach Anspruch 1, dadurch gekennzeichnet, dass die zweite Öffnung am Endbereich (54) eines sich in den Hohlraum des Hohlkörpers (42) erstreckenden Entlüftungsrohrs (50) vorgesehen ist.

3. Tropfkammer nach Anspruch 2, dadurch gekennzeichnet, dass der Endbereich (54) des Entlüftungsrohrs (50) mit einer Kappe (80) versehen ist, deren Öffnung (78) die Membran (56) überspannt, wobei die Membran (56) mit dem Umfangsrand der Öffnung (78) verbunden ist.

4. Tropfkammer nach Anspruch 2, dadurch gekennzeichnet, dass die Membran (56) den Endbereich (54) des Entlüftungsrohrs überspannt und auf dem Umfangsrand des Entlüftungsrohrs (50) befestigt ist.

5. Tropfkammer nach einem der Ansprüche 1 bis 4, dadurch gekennzeichnet, dass die Neigung zur Längsachse des Entlüftungsrohrs (50) 30 bis 60°, insbesondere etwa 45° beträgt.

6. Tropfkammer nach Anspruch 1, dadurch gekennzeichnet, dass die zweite Öffnung (88) in der Aussenwand des Hohlkörpers (42) vorgesehen ist.

7. Tropfkammer nach einem der Ansprüche 1 bis 6, dadurch gekennzeichnet, dass die Membran (56) aus einer Kunststoff-Folie mit mikroporöser Struktur besteht, wobei der Porendurchmesser dieser Kunststoff-Folie mit einer Dicke von 0,1 bis 0,8 mm höchstens 1 μm, insbesondere höchstens 0,5 μm beträgt.

8. Tropfkammer nach Anspruch 7, dadurch gekennzeichnet, dass der Membranwerkstoff PTFE, Silikon, Polyethylen, Polypropylen oder ein silikonisiertes Polymere ist.

9. Tropfkammer nach Anspruch 7 oder 8, dadurch gekennzeichnet, dass die Membran (56) auf einer polymeren Stützschicht vorgesehen und kammerseitig angeordnet ist.

## Claims

1. Drip chamber, especially for use in a haemodialysis apparatus, having a hollow body (42) with a floor (44) and a cover (38) with an inlet opening in the cover (38) to admit the liquid to be fed, a second opening (78, 88) for ventilation and means (56) to close the second opening (78, 88) with respect to the liquid and having an outlet opening (46) in the floor (44), characterised in that the second opening (78, 88) is inclined to the horizontal and that the means for closing this second opening (78, 88) is a liquid-impenetrable, gas permeable membrane (56) which covers the opening (78, 88).

2. Drip chamber according to claim 1, characterised in that the second opening is provided at its end region (54) with a ventilation tube (50) extending in to the interior of the hollow body (42).

3. Drip chamber according to claim 2, characterised in that the end region (54) of the ventilating tube (50) is provided with a cap (80), over the opening of which the membrane (56) is stretched, wherein the membrane (56) is connected to the periphery of the opening (78).

4. Drip chamber according to claim 2, characterised in that the membrane (56) stretches across the end region (54) of the ventilating tube and is fastened to the periphery of the ventilating tube (50).

5. Drip chamber according to any of claims 1 to 4, characterised in that the inclination to the lengthwise axis of the ventilating tube (50) is 30 to 60°, especially about 45°.

6. Drip chamber according to claim 1, characterised in that the second opening (88) is provided in the outer wall of the hollow body (42).

7. Drip chamber according to any of claims 1 to 6, characterised in that the membrane (56) comprises a synthetic film with a microporous structure, in which the pore diameter of this synthetic film having a thickness of 0.1 to 0.8 mm is at most 1 μm, especially at most 0.5 μm.

8. Drip chamber according to claim 7, characterised in that the membrane material is PTFE, silicon, polyethylene, polypropylene or a siliconised polymer.

9. Drip chamber according to claim 7 or 8, characterised in that the membrane (56) is provided on a polymeric supporting layer and arranged at the side of the chamber.

## Revendications

1. Chambre de goutte à goutte, utilisable en particulier dans un appareil d'hémodialyse, comprenant un corps creux (42) avec un fond (44) et un couvercle (38), une ouverture d'alimentation dans le couvercle (38) pour l'introduction du liquide à traiter, une deuxième ouverture (78, 88) pour la désaération et des dispositifs (56) pour fermer la deuxième ouverture (78, 88) pour le liquide, et un orifice d'écoulement (46) dans le fond (44), caractérisée en ce que la deuxième ouverture (78, 88) est en disposition inclinée par rapport à l'horizontale et en ce que le dispositif pour la fermeture de cette deuxième ouverture (78, 88) est une membrane perméable aux gaz mais refoulant les liquides (56) qui recouvre l'ouverture (78, 88).

2. Chambre de goutte à goutte selon la revendication 1, caractérisée en ce que la deuxième ouverture est prévue dans la région terminale (54) d'un tube de désaération (50) pénétrant dans l'espace intérieur du corps creux (42).

3. Chambre de goutte à goutte selon la revendication 2, caractérisée en ce que la région terminale (54) du tube de désaération (50) porte un capuchon (80) dont l'ouverture (78) est recouverte par la membrane (56) qui est fixée avec la périphérie de l'ouverture (78).

4. Chambre de goutte à goutte selon la revendication 2, caractérisée en ce que la membrane (56) recouvre la région terminale (54) du tube de désaération et est fixée sur le bord périphérique du tube de désaération (50).

5. Chambre de goutte à goutte selon l'une des revendications 1 à 4, caractérisée en ce que l'inclinaison sur l'axe longitudinal du tube de désaération (50) est de 30 à 60°, plus spécialement d'environ 45°.

6. Chambre de goutte à goutte selon la revendication 1, caractérisée en ce que la deuxième ouverture (88) est placée dans la paroi extérieure du corps creux (42).

7. Chambre de goutte à goutte selon l'une des revendications 1 à 6, caractérisée en ce que la membrane (56) consiste en une feuille de résine synthétique à structure microporeuse, le diamètre de pore de cette feuille de résine synthétique, d'une épaisseur de 0,1 à 0,8 mm, étant au maximum de 1 μm et plus spécialement au maximum de 0,5 μm.

8. Chambre de goutte à goutte selon la revendication 7, caractérisée en ce que la membrane consiste en PTFE, silicone, polyéthylène, polypropylène ou un polymère siliconé.

9. Chambre de goutte à goutte selon la revendication 7 ou 8, caractérisée en ce que la membrane (56) est appliquée sur une couche de support polymère et disposée du côté de la chambre.

0 062 913

_Fig. 1_

_Fig. 2_

_Fig. 2a_  _Fig. 2b_

7

**Fig. 3**

54

50

54

86

52

78

56

80

82

84

**Fig. 4**

54

50

54

86

52

56

84

**Fig. 5**

36  50  68

90  56

30

88

42